# EUROPEAN PATENT APPLICATION

(11) **EP 2 444 043 A1**
(43) Date of publication of application: **25.04.2012**
(21) Application number: 10447021.6
(22) Date of filing: 13.10.2010
(51) Int. Cl.: A61F 13/15

(54) **Improved absorbent structure**

(71) Applicant: Romanova bvba starter, 9255 Buggenhout (BE)
(72) Inventor: van de Maele, Marleen, 9255 Buggenhout (BE)

(57) **Abstract**

The present invention relates to an improved absorbent structure and to an improved absorbent article obtainable therewith, as well as to a method of obtaining the absorbent structure. The present invention is of importance in the field of personal hygiene. In particular, it is of interest for the manufacturing of absorbent articles such as feminine hygiene garments, baby diapers, baby pants, and adult incontinence garments. The absorbent structures according to the invention comprise a substantially liquid-impermeable wicking layer, an immobilisation layer which joins to the substantially liquid-impermeable wicking layer to define compartments there between; and an absorbent material held in at least one of the compartments. The present invention foresees in the need for improved thin, flexible, lightweight absorbent structure for use in an absorbent article which overcomes the absorbency problems of the prior art during absorption, distribution and retention of bodily exudates with optimal fit and wearing comfort.

## Description

### FIELD OF THE INVENTION

The present invention relates to an improved absorbent structure and to an improved absorbent article obtainable therewith, as well as to a method of obtaining the absorbent structure. The present invention is of importance for the field of personal hygiene. In particular, it is of interest for the manufacturing of absorbent articles such as feminine hygiene garments, baby diapers, baby pants, and adult incontinence garments.

### BACKGROUND OF THE INVENTION

Absorbent articles such as feminine hygiene garments, baby diapers, baby pants, and adult incontinence garments are well known consumer products. In their development, attempts are often targeted at improving the absorption efficiency of these products. Modern products make use of for instance absorbent polymer materials which in a typical baby diapers allow storing amounts as high as 300 ml of liquid.

In spite of the incorporation of absorbent polymer materials with improved liquid absorption capacity, their full potential often goes unexploited.

When a particle of the absorbent polymer material in an absorbent structure is wetted, it swells and forms a gel. This gel formation can block liquid transmission towards the interior of the absorbent layer, a phenomenon called "gel blocking". Gel blocking in and adjacent a zone of the absorbent layer of initial liquid contact prevents liquid from rapidly dispersing or wicking past the "blocking" material into the rest of the absorbent layer and further liquid uptake by the absorbent layer must then take place via a diffusion process that is much slower than the rate at which liquid is applied to the absorbent layer. The diminished capacity to absorb and distribute liquid into the absorbent articles results in leakages, well before the absorbent core is fully saturated.

The above problem has been addressed by not completely abandoning the use of hydrophilic fibrous materials next to the use of absorbent polymer materials. However, it will be clear that the absolute and relative proportions of the hydrophilic fibre and absorbent polymer materials will thereby be restricted in order to maintain the absorbent properties of the absorbent structure. Consequently, limits are imposed on reducing the amount of hydrophilic fibre and thus also on reducing the thickness of the absorbent structure.

Modern absorbent articles often comprise a non-woven layer upon which a layer of absorbent polymer material is provided. The absorbent polymer material layer is covered by an immobilisation layer attached to the non-woven layer so as to provide compartments comprising the absorbent polymer material. When this absorbent article becomes wet, the polymer materials swell and exert pressure on the immobilisation layer. As a consequence the immobilisation layer may tear fibres out of the non-woven layer and detach. The wet immobilisation, i.e. immobilisation of absorbent material when the article is wet or at least partially loaded, is consequently partially or fully lost.

Furthermore, the non-woven materials also contain cavities and spaces in between the fibers in which unbound quantities of liquid are often stored in stead of being distributed and transported to the absorbent components and/or absorbent elements from the absorbent structure. Such unbound or 'free' quantities of liquid run the risk of being pushed from the non-woven material up to the surface of the absorbent article, e.g. by movement of the wearer, and may give rise to absorbent article failure, leakages and/or surface wetness such a rewetting.

Absorbent articles have been devised wherein the absorbent polymer material is provided in particle form. To keep the absorbent particulate polymer material in their intended position, both when the absorbent article is dry as well when the absorbent article is wet, a large number of discrete compartments is provided. However, liquid entering the absorbent article on the seams in between the compartments is not passing by the high capacity absorbent polymer particles, leaving substantial amounts of absorbent capacity unexploited.

In view of the above, in particular when the absorbent structure for an absorbent article is to absorb high amounts of liquids, the absorption, distribution, retention and wet immobilisation may not be fully satisfactory.

Despite the development of absorbent structures, there remains a need for improved absorbent structures which can adequately reduce the incidence of leakage from absorbent articles, such as disposable diapers. There is a need for an absorbent structure which can provide improved handling of liquid and more effectively uptake and retain loadings of liquid during use.

In particular, the present invention aims to provide an improved absorbent structure which provides increased absorption speed and improved fluid distribution and transport allowing an improved usage of the absorbent materials available within the absorbent article. The present invention further aims to provide an absorbent article in which the absorbent polymer material is incorporated in such a form, position and/or manner as to provide increased user comfort. The present invention also aims to provide an absorbent structure for use in an absorbent article which provides fewer leakages, better fit and is thinner, more flexible, discreet and/or form matching.

### SUMMARY OF THE INVENTION

The present invention relates to an absorbent structure for use in an absorbent article. In particular the present invention provides an absorbent structure as described by claim 1. In particular, the present invention provides an absorbent structure for use in an absorbent article comprising:
a) a substantially liquid-impermeable wicking layer and
b) an immobilisation layer which is joined to the substantially liquid-impermeable wicking layer to define compartments there between; and
c) an absorbent material held in at least one of the compartments:
   i) wherein said absorbent material comprises an absorbent polymer material, and
   ii) from zero to an amount less than about 40 weight percent absorbent fibrous material, based on the weight of absorbent polymer material.

The absorbent structure is characterized by a wicking layer which is substantially liquid-impermeable on which liquids such as water, urine and/or other bodily exudates easily spread out. This has for effect that the liquid will distribute and transport more efficiently and effectively over the liquid-impermeable wicking layer so as to wet the side and lower sides of the absorbent polymer materials. The absorbent polymer material's absorption sites will be exposed more rapidly and evenly due to this increased liquid transport effect and will lead to improved utilisation of the absorbent materials in partially or fully bodily exudates loaded state.

The substantially liquid-impermeable wicking layer furthermore prevents unbound quantities of liquids from being imbibed, captured and/or stored in any cavities located below the substantially liquid-impermeable permeable wicking layer such as for instance present in some of the absorbent components or absorbent elements. Unbound 'free' quantities of liquid are at risk of being pushed out from said lower part of the absorbent structure to the surface of the absorbent article such as the top sheet, e.g. by movement of the wearer, and may give rise to product failure, leakage and/or surface wetness. In a preferred embodiment of this invention, the substantially liquid-impermeable permeable wicking layer has a contact angle with a water droplet lying thereon of less than 30°.

Hence, the substantially liquid-impermeable wicking layer according to an embodiment of the invention with related substantially liquid-impermeable wicking capabilities allows the absorbent structure to improve absorption, distribution and retention of the liquids while reducing the leakage and surface wetness of the absorbent products containing such absorbent structures. The capacity of the absorbent polymer materials to take up liquid is exploited more fully, and due to quick transport and mass flow of the unbound amounts of liquids from the different locations of the non-absorbent liquid-impermeable wicking layer to the absorbent structure, elements and components where the liquid can be absorbed and bound results in overall low rewet values, less leakage risk and less surface wetness and thus increase reliability of the overall absorbent article. The present invention furthermore provides an environmentally-friendly absorbent article which is capable of reducing the total amount of raw materials by improved utilisation of the raw materials available, and furthermore limits energy consumption.

In a further aspect, the present invention provides an absorbent article, as provided by claim 11, comprising at least in the front half of the absorbent article, an absorbent structure according to an embodiment of the invention.

An absorbent article according to an embodiment of the invention has the advantage that the risk of leaks is reduced. The fit, reliability and comfort level provided is also improved, while the thinner absorbent articles increase discreetness and noise levels. In a third aspect, the present invention provides a method for the manufacturing of an absorbent structure for use in an absorbent article as provided by claim 14. In particular, the present invention provides a method for the manufacturing of an absorbent structure for use in an absorbent article comprising the steps of:
- providing a substantially liquid-impermeable wicking layer,
- covering the substantially liquid-impermeable wicking layer with an absorbent material wherein said absorbent material comprises
   i) an absorbent polymer material, and
   ii) from zero to an amount less than about 40 weight percent absorbent fibrous material, based on the weight of absorbent polymer material,
- covering the absorbent material with an immobilisation layer which is joinable to the substantially liquid-impermeable wicking layer, and
- joining the immobilisation layer to the substantially liquid-impermeable wicking layer to define compartments there between wherein the absorbent material is held in at least one of the compartments.

A method according to an embodiment of the invention has the advantage that better performing absorbent articles can be produced.

Unlike previously existing absorbent articles and methods relating thereto limited by the relative concentrations of the absorbent polymer material and the hydrophilic fibre material to still enable them to absorb liquid at the volume and rate at which the bodily exudates are applied to the absorbent article during use, the present invention overcomes the situation by increased fluid communication and management in the form of spreading, dispersing and transporting the liquids from the less absorbent area's of the absorbent material layer to the more absorbent area's of the absorbent material layer resulting in reduced leakages and low rewet values.

Further advantageous features, aspects and details of the invention are evident from the dependent claims, the description and drawings as explained in more detail down below.

### BRIEF DESCRIPTION OF FIGURES

**Figure 1** provides a cross-sectional schematic illustration of an absorbent structure according to an embodiment of the invention.
**Figure 2** provides a cross-sectional schematic illustration of an absorbent structure according to another embodiment of the invention.
**Figure 3** provides a cross-sectional schematic illustration of an absorbent structure according to another embodiment of the invention.
**Figure 4** provides a top view schematic illustration of different area's of junction patterns of absorbent structures according to embodiments of the invention.
**Figure 5** provides a top view schematic illustration of different clusters of absorbent material of absorbent structures according to embodiments of the invention.
**Figure 6** is a top plan view of a diaper as a preferred embodiment of an absorbent article according to the invention, with the upper layers partially cut away.

### DETAILED DESCRIPTION OF THE INVENTION

The present invention concerns an improved absorbent structure for use in an absorbent article, preferably a disposable one, such as feminine hygiene garments, baby diapers and pants and adult incontinence garments; and to a method and manufacturing of the same.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, insofar such variations are appropriate to perform in the disclosed invention. However, it is to be understood that the value to which the modifier "about" refers is itself also specifically disclosed.
"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.
"Absorbent core" as used herein refers to a three-dimensional part of the absorbent structure, comprising liquid-absorbing material, useful to absorb and/or retain bodily exudates.
"Absorbent component" as used herein refers to a structural constituent of an absorbent structure, e.g., a piece of an absorbent core, such as one of multiple pieces in a multi-piece absorbent core.
"Absorbent element" as used herein refers to a part of a functional constituent of an absorbent structure, e.g., a liquid acquisition layer, a liquid distribution layer, or a liquid storage layer formed of a material or materials having particular liquid handling characteristics suitable for the specific function.
"Absorbent insert" as used herein refers to a device adapted for insertion into an absorbent article and to serve as an absorbent structure when so inserted.
"Absorbent layer" as used herein refers to a term referring to a discrete, identifiable sheet-like or web-like element of an absorbent structure which may remain detached and relatively movable with respect to another such element or may be attached or joined so as to remain permanently associated with another such element. Each absorbent layer may itself include a laminate or combination of several layers, sheets and/or webs of similar or diverse compositions.
"Absorbent polymer material", "absorbent gelling material", "AGM", "superabsorbent", "superabsorbent material", "super absorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01).
"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g. bond area's) or unintentional (e.g. manufacturing artefacts).
"Absorbent particulate polymer material" as used herein refers to an absorbent polymer material which is in particulate form such as powders, granules, flakes and the like so as to be flowable in the dry state.
"Absorbent particulate polymer material area" as used herein refers to the area of the absorbent structure wherein adjacent layers are separated by a multiplicity of absorbent polymer material. Incidental contact areas between these adjacent layers within the absorbent particulate polymer material area may be intentional or unintentional (e.g. manufacturing artefacts).
"Absorbent structure" as used herein refers to those elements of an absorbent article comprising material or a combination of materials suitable to absorb, distribute and retain bodily exudates.
"Absorption" as used herein refers to the process by which a liquid is taken up within a material.
"Acquisition layer", "acquisition region", "acquisition surface" or "acquisition material" and the like as used herein refer to a layer having a faster liquid uptake capability.
"Absorbency" is the ability of a material to take up fluids by various means including capillary, osmotic, solvent, chemical or other action.
"Adult incontinence garment" as used herein refers to absorbent articles intended to be worn by incontinent adults, for absorbing and containing bodily exudates.
"Adhesion" as used herein refers to the force that holds different materials together at their interface.
"Adhesive" as used herein refers to a material, which may or may not be flowable in solution or when heated, that is used to bond materials together.
"Adsorption" as used herein refers to the process by which a liquid is taken up by the surface of a material.
"Air laying" as used herein refers to forming a web by dispersing fibres or particles in an air stream and condensing them from the air stream onto a moving screen by means of a pressure or vacuum; a web of fibres produced by air laying is herein referred to an "airlaid"; an airlaid web bonded by one or more techniques to provide fabric integrity is herein referred to an "airlaid nonwoven".
"Apparent density" as used herein refers to the basis weight of the sample divided by the calliper with appropriate unit conversions incorporated therein. Apparent density used herein has the unit g/cm³.
"Attach", "attached" and "attachment" as used herein are synonymous with their counterparts of the terms "fasten", "affix", "secure", "glue", "bind", "join" and "link".
"Baby diaper" as used herein refers to absorbent articles intended to be worn by children, for absorbing and containing bodily exudates which the user draws up between the legs and fastens about the waist of the wearer.
"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.
"Back region" as used herein refers to the portion of an absorbent article or part thereof that is intended to be positioned proximate the back of a wearer.
"Backing" as used herein refers to a web or other material that supports and reinforces the back of a product.
"Basis weight" is the weight per unit area of a sample reported in grams per square meter or g/m²_{.}
"Bodily exudates", "body exudates", "bodily fluids", "body fluids", "bodily discharges", "body discharges", "liquids" and the like as used herein are used interchangeably and refer to, but are not limited to urine, blood, vaginal discharges, breast milk, sweats and faecal matter.
"Binder", "adhesive", "glue", "resins", "plastics" and the like as used herein are used interchangeably and refer to substances, generally in a solid form (e.g. powder, film, fibre) or as a foam, or in a liquid form (e.g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.
"Bond strength" as used herein refers to the amount of adhesion between bonded surfaces. It is a measure of the stress required to separate a layer of material from the base to which it is bonded.
"Capillary action", "capillarity", or "capillary motion" and the like as used herein are used to refer to the phenomena of the flow of liquid through porous media.
"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.
"Cellulose fibres" as used herein refers to naturally occurring fibres based on cellulose, such as, for example cotton, linen, etc; wood pulp fibres are one example of cellulose fibres; man-made fibres derived from cellulose, such as regenerated cellulose (rayon), or partially or fully acetylated cellulose derivatives (e.g. cellulose acetate or triacetate) are also considered as cellulose fibres.
"Cluster' or the like as used herein refers to an agglomeration of particles and/or fibres.
"Chemically stiffened fibers", chemically modified fibers", "chemically cross-linked fibers", "curly fibers" and the like as used herein are used interchangeably and refer to any fibers which have been stiffened by chemical means to increase stiffness of the fibers under both dry and aqueous conditions, for example by way of addition of chemical stiffening agents (e.g. by coating, impregnating, etc), altering the chemical structure of the fibers themselves (e.g. by cross-linking polymer chains, etc) and the like.
"Cohesion" as used herein refers to the resistance of similar materials to be separated from each other.
"Compartment" as used herein refers to chambers, cavities, pockets and the like.
"Comprise," "comprising," and "comprises" and "comprised of" as used herein are synonymous with "include", "including", "includes" or "contain", "containing", "contains" and are inclusive or open-ended terms that specify the presence of what follows e.g. a component and do not exclude or preclude the presence of additional, non-recited components, features, elements, members, steps, known in the art or disclosed therein.
"Coverstock" as used herein refers to a lightweight non-woven material used to contain and conceal an underlying absorbent core material; examples are the facing layer or materials that cover the absorbent cores of feminine hygiene garments, baby diapers and pants and adult incontinence garments.
"Crotch region" of an absorbent article as used herein refers to about 50% of the absorbent article's total length (i.e., in the y-dimension), where the crotch point is located in the longitudinal centre of the crotch region. That is, the crotch region is determined by first locating the crotch point of the absorbent article, and then measuring forward and backward a distance of 25% of the absorbent article's total length.
"Cross direction (CD)", "lateral" or "transverse" and the like as used herein are used interchangeably and refer to a direction which is orthogonal to the longitudinal direction and includes directions within ±45° of the transversal direction.
"Curing" as used herein refers to a process by which resins, binders or plastics are set into or onto fabrics, usually by heating, to cause them to stay in place; the setting may occur by removing solvent or by cross-linking so as to make them insoluble.
"Diaper", "conventional diaper", "diaper-like", "diaper-like garment" and the like as used herein are used interchangeably and refer to disposable absorbent articles, which typically include a front waist portion and a back waist portion which may be releasably connected about the hips of the wearer during use by conventional fasteners such as adhesive tape fasteners or hook and loop type fasteners. In use, the article is positioned between the legs of the wearer and the fasteners are releasably attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.
"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).
"Distribution layer", "distribution region", "distribution surface" or "distribution material" and the like as used herein are used interchangeably and refer to a layer having a larger capacity in wicking, dispersing and distributing liquids.
"Dry laying" as used herein refers to a process for making a non-woven web from dry fibre; these terms apply to the formation of carded webs, as well as to the air laying formation of random webs; a web of fibres produced by dry laying is herein referred to as a "drylaid"; a drylaid web bonded by one or more techniques to provide fabric integrity is herein referred to a "drylaid nonwoven".
"Feminine hygiene garments" as used herein refer to absorbent hygiene articles intended to be worn by woman, for absorbing and containing body exudates.
"Fibre" as used herein refers to the basic threadlike structure from which non-woven, yarns and textiles are made. It differs from a particle by having a length at least 4 times its width; "Natural fibres" are either of animal (wool, silk), vegetable (cotton, flax, jute) or mineral (asbestos) origin, while "Man-made fibres" may be either polymers synthesised from chemical compounds (polyester, polypropylene, nylon, acrylic etc.) or modified natural polymers (rayon, acetate) or mineral (glass). "Fibre" and "filament" are used interchangeably.
"Dry strength" as used herein refers to the strength of an adhesive joint determined in dry state conditions, immediately after drying under specified conditions or after a period of conditioning in the standard laboratory atmosphere.
"Fabric" as used herein refers to a sheet structure made from fibres, filaments and/or yarns.
"Film", "foil" and the like as used herein are used interchangeably and refer to a thin sheet of substantially liquid-impermeable material such as plastic or closed foams. In this invention it particularly refers to materials that do not correspond to non-woven.
"Fluff pulp" as used herein refers to wood pulp specially prepared to be dry laid.
"Front region" as used herein refers to the portion of an absorbent article or part thereof that is intended to be positioned proximate the front of a wearer.
"Garment facing layer' as used herein refers to elements of the chassis that form the outer surface of the absorbent article, such as the back sheet, the side panels, the waist fasteners, and the like, when such elements are present.
"Heat activated adhesive" as used herein refers to a dry adhesive that is rendered tacky or fluid by application of heat or heat and pressure to the assembly.
"Heat sealing adhesive" as used herein refers to a thermoplastic adhesive which is melted between the adherent surfaces by heat application to one or both of the adjacent adherent surfaces.
"Highloft" as used herein refers to general term of low density, thick or bulky fabrics.
"Hot-melt adhesive" as used herein refers to a solid material that melts quickly upon heating, then sets to a firm bond upon cooling; used for almost instantaneous bonding.
"Hydrophilic" as used herein refers to having an affinity for being wetted by water or for absorbing water.
"Hydrophobic" as used herein refers to lacking the affinity for being wetted by water or for absorbing water.
"Immobilisation layer" as used herein refers to a layer able to be applied to the absorbent polymer material with the intent to bond and/or immobilize absorbent material.
"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.
"Knitting" as used herein refers to the technique for interlocking loops of fibres with needles or similar devices.
"Layer" refers to identifiable components of the absorbent article, and any part referred to as a "layer" may actually comprise a laminate or combination of several sheets or webs of the requisite type of materials. As used herein, the term "layer" includes the terms "layers" and "layered." "Upper" refers to the layer of the absorbent article which is nearest to and faces wearer-facing layer; conversely, the term "lower" refers to the layer of the absorbent article which is nearest to and faces the garment-facing layer. "Layer" is three dimensional structure with a x dimension width, y dimension length, and z-dimensions thickness or calliper, said x-y dimensions being substantially in the plane of the article, however it should be noted that the various members, layers, and structures of absorbent articles according to the present invention may or may not be generally planar in nature, and may be shaped or profiled in any desired configuration.
"Machine direction (MD)", "longitudinal" and the like as used herein are used interchangeably and refer to a direction running parallel to the maximum linear dimension of the structure and includes directions within ±45° of the longitudinal direction.
"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.
"Mass flow" as used herein refers to the flow of a liquid from one absorbent element or component to another absorbent element or component by channel flow action.
"Mechanical bonding" as used herein refers to a method of bonding fibres by entangling them. This can be achieved by needling, stitching with fibres or by the use of high-pressure air or water jets and the like.
"Non-woven" as used herein refers to manufactured sheet, web or batt of directionally or randomly orientated fibres, bonded by friction, and/or cohesion and/or adhesion, excluding paper and products which are woven, knitted, tufted, stitch-bonded incorporating binding yarns or filaments, or felted by wet-milling, whether or not additionally needled. The fibres may be of natural or man-made origin and may be staple or continuous filaments or be formed in situ. Commercially available fibres have diameters ranging from less than about 0.001 mm to more than about 0.2 mm and they come in several different forms: short fibres (known as staple, or chopped), continuous single fibres (filaments or monofilaments), untwisted bundles of continuous filaments (tow), and twisted bundles of continuous filaments (yarn). Non-woven fabrics can be formed by many processes such as melt blowing, spun bonding, solvent spinning, electro spinning, and carding. The basis weight of non-woven fabrics is usually expressed in grams per square meter (gsm).
"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened).
"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.
"Rear" as used herein refers to the portion of an absorbent article or part thereof that is intended to be positioned proximate the back of the wearer.
"Resin" as used herein refers to a solid or semisolid polymeric material.
"Substantially cellulose free" as used herein refers to an absorbent article, structure or core, that contains less than 40% by weight cellulosic fibers, less than 20% cellulosic fibers, less than 5% cellulosic fibers, no cellulosic fibers, or no more than an immaterial amount of cellulosic fibers which do not materially affect the thinness, flexibility or absorbency thereof.
"Thermobonding" as used herein refers to a method of bonding fibres by the use of heat and/or high-pressure.
"Thermoplastic" as used herein refers to polymeric materials that have a melting temperature and can flow or be formed into desired shapes on the application of heat at or below the melting point.
"Ultrasonic" as used herein refers to the use of high frequency sound to generate localised heat through vibration thereby causing thermoplastic fibres to bond to one another.
"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.
"Wearer facing layer" as used herein refers to elements of the chassis that form the inner surface of the absorbent article, such as the topsheet, the leg cuffs, and the side panels, etc., when such elements are present.
"Weaving" as used herein refers to the process of interlacing two or more sets of yarns at right angles to form a fabric; a web of fibres produced by weaving is herein referred to as a "Woven".
"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (the y-direction) will be significantly larger than the thickness, but less than the length. Often, though not necessarily, the thickness and the width of such materials is essentially constant along the length of the web. Without intending any limitation, such web materials may be cellulosic fiber materials, tissues, woven or non-woven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, non woven / film laminates. Web materials may comprise other materials, such as added binding material, particles, hydrophilizing agents and the like.
"Wet burst strength" is a measure of a layer's ability to absorb energy, when wet and subjected to deformation normal to the plane of the web.
"Wet strength" as used herein refers to the strength of an adhesive joint determined immediately after removal from a liquid in which it has been immersed under specified conditions of time, temperature and pressure. The term is commonly used in the art to designate strength after immersion in water.
"Wet laying" as used herein refers to the forming a web from an aqueous dispersion of fibres by applying modified paper making techniques; a web of fibres produced by wet laying is herein referred to as a "wetlaid".
"Wicking" or the like, as used herein refers to the flow of a liquid by capillary transport through porous media, by mass flow and/or by transport, due to gravitational, movement or other forces, of liquid over a substantially liquid-impermeable surface.
"Wood pulp" as used herein refers to cellulosic fibres used to make viscose rayon, paper and the absorbent cores of products such as feminine hygiene garments, baby diapers and pants and adult incontinence garments.
"X-y dimension" as used herein refers to the plane orthogonal to the thickness of the article, structure or element. The x- and y-dimensions correspond generally to the width and length, respectively, of the article, structure or element.
"Z-dimension" as used herein refers to the dimension orthogonal to the length and width of the article, structure or element. The z-dimension corresponds generally to the thickness of the article, structure or element.

The recitation of numerical ranges by endpoints includes all numbers and fractions subsumed within that range, as well as the recited endpoints.

The present invention has resulted from a realization that an absorbent article having a liquid-permeable top sheet, a liquid-impermeable back sheet, and an absorbent structure, wherein the absorbent structure comprises a substantially liquid-impermeable wicking layer, an immobilisation layer joined to the substantially liquid-impermeable wicking layer to define compartments there between and wherein absorbent material is held in at least one of the compartments, can be more efficiently used to absorb, disperse and transport a liquid, such as urine, if the absorbent structure is provided with a substantially liquid-impermeable wicking layer thereby avoiding leakages and high rewet values by preventing unbound liquids in between the compartments and by dispersing the liquid permeating within the absorbent structure from the less absorbent area's (e.g. saturated) to the more absorbent area's (e.g. unsaturated) by various liquid flow actions.

In particular, the invention provides an absorbent structure for use in an absorbent article comprising:
a) a substantially liquid-impermeable wicking layer and
b) an immobilisation layer which is joined to the substantially liquid-impermeable wicking layer to define compartments there between; and
c) an absorbent material held in at least one of the compartments:
   i) wherein said absorbent material comprises an absorbent polymer material, and
   ii) from zero to an amount less than about 40 weight percent absorbent fibrous material, based on the weight of absorbent polymer material.

A preferred embodiment of the absorbent structure according to the present invention is characterized by a substantially liquid-impermeable wicking layer having a contact angle less than about 30 °, indicating that the layer is hydrophilic.

The contact angle is a measure of hydrophilicity. A water droplet lying on a layer of material which is hydrophobic, will display a large contact angle. The more hydrophilic the layer is, the smaller the contact angle. The contact angle of a water droplet can be measured using techniques well-known to a person skilled in the art. The contact angle α with respect to water is measured and used as a measure of the level of hydrophilic properties of a coated or uncoated film or foil surface. The smaller the contact angle, the greater is the level of hydrophilic properties. The measurement can for instance be carried out on a G1 goniometer, available from Krüss, Hamburg, Germany.

The above described absorbent structure comprising an hydrophilic substantially liquid-impermeable wicking layer has for effect that liquid which comes in contact with the substantially liquid-impermeable wicking layer will readily spread out over said surface. Since upon said spreading out the liquid will touch at least one or more of the absorbent materials, such as for instance absorbent polymer materials, located at the adjacent and lower surface of the absorbent polymer material area, being the side opposite the major surface side where the liquid was first deposited. The distribution, transport and absorption of the liquid is thus greatly improved and this speedy distribution will help to avoid large amounts of unbound quantities of liquid within the absorbent structure preventing absorbent article failure and high rewet values. Another preferred embodiment of an absorbent structure according to the present invention is characterized by a substantially liquid-impermeable wicking layer which is hydrophobic. Since the larger globe-like droplets provided on such hydrophobic structures have increased capabilities to be roll, move and be transported on the substantially liquid-impermeable wicking layer via gravitational, movement and other forces the dispersion is also increased in these absorbent structures.

Certainly combinations of both hydrophilic and hydrophobic regions, area's and grids specifically depending on their structural and/or functional location within the absorbent structure are hereby also encompassed.

In the present invention the term "wicking layer" refers to a preferably hydrophilic or hydrophobic layer and combinations thereof beneath the absorbent material. Due to its position and technical features, liquid will easily spread out on its surface, consequently the liquid is redirected to the absorbent material there where it can be absorbed.

The substantially liquid-impermeable wicking layer according to the present invention ensures enhanced fluid wicking, dispersion and/or transport throughout the absorbent material layer by more closely connecting the compartments comprising absorbent polymer materials and enhancing liquid communication. Additionally the more intense contact in between the lower surface of the absorbent polymer material and the upper surface of the wicking medium allows faster and more complete absorption, adsorption and distribution in between the individual absorbent polymer compartments who are in the prior art typically physically and/or chemically cut off from one another for instance by thermo-pressure bonding, heat sealing adhesive, adhesive pockets, etc, by providing an additional and more direct co-extensive wicking, dispersion and transportation of liquid to the still more absorbent underused compartments and components of the absorbent structure increasing effectiveness and efficiency of the available absorbing raw materials. The presence of unbound liquid droplets leading to leakages, high rewet values and discomfort are thereby minimized.

In a preferred embodiment of the absorbent structure of the invention, the substantially liquid-impermeable wicking layer is provided by a substantially continuous layer of foil, film, closed foam, plastic or similar substantially liquid-impermeable materials, media and/or layers.

The effect of providing the substantially liquid-impermeable wicking layer in the form of a substantially continuous layer of film or foil is that a substantially impervious barrier is provided. The provision of a film or foil is advantageous compared to the use of a pervious layer, such as for instance a non-woven material, as in contrast to such non-woven material the film or foil is not comprised of fibers. Hence, it is not possible that fibers are pulled out upon the exertion of a force onto the film or foil, for instance during movement and/or liquid uptake process (e.g. swelling and expansion of the absorbent material such as super absorbent polymers). In a preferred embodiment this ensures the bonds, joints and/or connections between the garment facing surface of the immobilization layer and the wearer facing surface of the wicking medium do not unintentionally detach during the dry state and/or wetting process. Furthermore, non-woven materials contain open spaces and cavities in between the fibers in which unbound quantities of liquid may be stored, which may lead to an increased risks of leakages and augmented rewet with reduced comfort and fit. Therefore according to an embodiment of the invention the absorbent material contained within the absorbent structure is mainly supported by a film or foil lacking such open spaces or cavities having the capacity to bond droplets of liquids, thereby leading to more advantageous and beneficial absorbent articles. Obviously besides the herein disclosed wicking medium material, other materials having these or similar characteristics known to the persons skilled in the art can also be used to perform this function.

In a preferred embodiment of the absorbent structure according to the invention, the film or foil is hydrophilic. In a more preferred embodiment, it is provided with a hydrophilic coating. Combinations thereof are hereby also encompassed.

The effect of providing the film or foil with a hydrophilic coating is that the properties of the film or foil can be modified according to the intended use. For instance, synthetic materials that are inherently hydrophobic such as polyethylene (PE), polypropylene (PP) or polyester terephtalate (PET) can be provided with a coating layer such that the resulting material has a contact angle of less than 50°, preferably less than 35°, more preferably less than 25°, still more preferably less than 20°, most preferably less than 15°, for a water droplet lying thereon. An advantage may also be formed in the fact that a cheap base layer, itself not displaying the desired property of readily spreading out liquid droplets over its surface, can be adjusted on at least one of its sides to display the desired property. Cost savings can be provided. A manufacturer can for instance purchase a film, foil, foam or plastic in bulk quantities and modify it with in-house recipes to the desired performance. Obviously such coating can be applied continuously, substantially continuously, partially or at discreet locations possibly showing multiple flow enhancing patterns and/or shapes depending on the desired end product and features.

In another preferred embodiment of the absorbent structure according to the invention, the film or foil is hydrophobic. In a more preferred embodiment, it is provided with a hydrophobic coating.

The effect of providing the film or foil with a hydrophobic coating is that the properties of the film or foil can be modified according to the intended use. For instance, synthetic materials that are inherently hydrophilic can be provided with a hydrophobic coating layer such that the resulting material shows hydrophobic properties. Here also a cheap base layer, itself not displaying the desired properties, can be adjusted on at least one of its sides to display the desired property. Cost savings can be provided. A manufacturer can for instance purchase a foil, film or plastic in bulk quantities and modify it with in-house recipes to the desired performance. Obviously such coating can be applied continuously, substantially continuously, partially or at discreet locations possibly showing multiple flow enhancing patterns and/or shapes depending on the desired end product.

In a preferred embodiment of the absorbent structure of the invention, the film or foil has on both of its surfaces, running parallel with the longitudinal axis of the film or foil, a contact angle with a water droplet lying thereon of less than 50°, preferably less than 35°, more preferably less than 25°, still more preferably less than 20°, most preferably less than 15°. Hence, the film or foil is hydrophilic on its top side as well as bottom side. A film or foil with this property can advantageously be used as a substantially liquid-impermeable wicking layer sandwiched between two layers of absorbent material, in particular one layer provided on the top side of the film or foil and the second provided on the bottom side of the film or foil. Although embodiments according to the present inventions showing hydrophilic wicking media are preferred, alternatively, wicking films or foils which are for instance partially, substantially or fully hydrophobic on both sides are also envisaged.

In a preferred embodiment of the absorbent structure of the invention, the film or foil is provided with access means, such as perforations, holes, channels, openings, compartmentalisation and the like, to a second layer of absorbent material. The dimensions of these access means are such that a liquid present on the film or foil will only drain through the access means upon reaching a pre-determined limit. This has for effect that the film or foil can be provided with a spill-over effect. If the speed and/or capacity of absorption of by the first layer of absorbent material is insufficient and the liquid is not being sufficiently fast removed from the film or foil, it shall drain through the access means to a second layer of absorbent material. The second layer of absorbent material can provide additional storage capacity. It can furthermore avoid that the immobilisation layer joined to the substantially liquid-impermeable wicking layer becomes too wet, thereby additionally reducing the risk to leakage, high rewet values and failure of the absorbent article. A critical reduction of bonding strength which might lead to the unintentional release of the immobilisation layer from the substantially liquid-impermeable wicking layer can be avoided, thereby ensuring the immobilisation and/or restraining of the absorbent materials on their intended location to ensure sufficient absorbency. Moreover high liquid retention values and increased comfort and fit can be achieved hereby.

Suitable substantially liquid-impermeable wicking layers for use in the invention are for instance coated polyethylene or polypropylene films. A preferred substantially liquid-impermeable wicking layer would for instance be a corona-treated, hydrophilic, micro-embossed PE film in the 15-22 µm thickness range.

In preferred embodiment the substantially liquid-impermeable wicking layer comprises a polymer film, preferably a polyethylene (PE) film, which has a hydrophilic coating on at least one surface. The resulted coated polyethylene (PE) film has a high level of hydrophilic properties.

In a preferred embodiment of the absorbent structure of the invention, the immobilisation layer is a thermoplastic adhesive. Use of a thermoplastic adhesive as the immobilisation layer is advantageous as the material is easily sealable to a film or foil. Alternatively, the immobilisation layer is made from a paper, tissue, woven, knitted or non-woven layer, capable of immobilising the absorbent material in the compartments or pockets formed in between said immobilisation layer and the substantially liquid impermeable wicking layer.

Typically the functional and structural integrity of the substantially liquid-impermeable wicking layer allows sufficient strength to prevent the breaking out and migration from the absorbent material and/or absorbent polymer material into the surrounding areas of the absorbent core, thus leading to less performing absorbent polymer materials.

The substantially liquid-impermeable wicking layer according to an embodiment of the invention acts as an additional fluid management structure, spreading the liquid more effectively and efficiently over a larger surface of the absorbent polymer material, so that more absorbent polymer material is faster and more continuously exposed to the liquid to be absorbed.

In a preferred embodiment of the invention the material properties of the substantially liquid-impermeable wicking layer typically also allow for at least partial retraction of the wicking material in between the absorbent polymer material area during the initial swelling of the absorbent polymer material by bringing the forces exerted upon the immobilizing layer by the swelling of the absorbent polymer material to the connections between the substantially liquid-impermeable wicking layer and the immobilizing layer. In a further preferred embodiment, the connections are maintained during partial loading of the absorbent polymer material within the compartments while these bonds may become partially or completely detached in fully bodily exudates loaded state. In this way initial contact is maintained until swelling forces take the upper hand. This debonding is preferably only partial, still ensuring structural integrity, however not restraining the swelling of the absorbent polymer material so as to decrease liquid absorption and retention capacity.

According to the present invention the immobilisation material is a layer, fabric or composition, such as for example a high-loft, non-woven or paper, which immobilises and/or restrains the absorbent material in dry, substantially and/or completely wet state, by way of compartmentalising clusters of absorbent material between itself and the substantially liquid-impermeable wicking layer. In a preferred embodiment this immobilisation material can also be an adhesive in combination with other immobilisation diluents such as tackifying resins, plasticizers and additives such as antioxidants, but could well be any other composition able to substantially immobilise absorbent polymer material within the absorbent structure of the absorbent article. Some immobilisation materials are thermoplastic others are not, while some initially thermoplastic materials may lose their thermoplastic behaviour due to a curing step, e.g. initiated via heat, UV radiation, electron beam exposure or moisture or other means of curing, leading to the irreversible formation of a cross-linked network of covalent bonds. Those materials having lost their initial thermoplastic behaviour are herein also understood as immobilisation materials.

In a preferred embodiment of the absorbent structure of the invention, the immobilisation layer bonded to the substantially liquid-impermeable wicking layer has a dry bond strength which is higher than 0,05 N/cm, more preferably higher than 0,2 N/cm, even more preferably higher than 2,5 N/cm and most preferably higher than 5 N/cm. The preferred absorbent structure provides a wet burst strength in line with the intended use, and can be customised by the various described herein and those know in the art.

It has been found that immobilisation compositions most useful for immobilizing absorbent polymer materials are those which combine controlled cohesion and adhesion behaviour. Adhesion is critical to ensure that the immobilisation layer maintains sufficient contact with the absorbent polymer material and in particular with the wicking medium. Cohesion ensures that the bond or joint does not break, in particular in response to external forces, and namely in response to strain. The bond or joint is subject to external forces during usage and when the absorbent structure has acquired liquid, which is then stored in the absorbent polymer material which in response swells. A preferred bond or joint will allow for such swelling, without breaking and without imparting too many compressive forces, which would restrain the absorbent polymer material from swelling, but allowing the debonding from the wicking medium at discreet contact points to allow further controlled swelling, and allowing eventual release during too high compressive forces if necessary, thereby also ensuring flexibility, fit and comfort.

The absorbent material used for covering the substantially liquid-impermeable wicking layer comprises an absorbent polymer material. Optionally part of the absorbent material is provided in the form of absorbent fibrous material. Suitable absorbent fibrous materials are for example airlaid material, fibrous cellulose material.

In a preferred embodiment of the absorbent structure of the invention, the absorbent material comprises a super absorbent polymer (SAP), preferably the super absorbent polymer is present in the absorbent material in an average basis weight of least 100 g/m², preferably at least 200, more preferably at least 400, even more preferably at least 600, most preferably at least 700 g/m2. Use of a super absorbent polymer as absorbent material provides an improved absorption capacity.

The absorbent structure of the present invention has a low flexure-resistance. The flexure-resistance of the absorbent structures is measured by the Peak Bending Stiffness as determined by the test which is modelled after the ASTM D 4032-82 Circular Bend Procedure, the procedure being considerably modified and performed as described in EP0336578B1, hereby incorporated by reference. In a preferred embodiment of the absorbent article of the invention, the absorbent structure has a flexure-resistance of less than 500.0 grams, more preferably less than about 250.0-350.0 grams, and still more preferably less than about 175.0 grams and most preferably less than about 130.0 or 100.0 grams. Thus, the absorbent structure of the present invention is highly flexible and conforms very well to the various shapes of the urogenital region. Use of an absorbent structure with this low a flexure-resistance has for effect that an easily flexible structure can be provided. This feature is advantageous as such a structure will easily ply and allow the structure to follow a body shape. Consequently, improved wearer fit and comfort is provided.

The absorbent structure of the present invention has minimum wet immobilisation values as measured by the shaker test weight retention as determined by the Wet Immobilisation test as described in US Patent Application 20070167928, hereby incorporated by reference. In a preferred embodiment of the absorbent article of the invention, the absorbent structure has a wet immobilization of more than 50 wt%, preferably more than 70 wt%, more preferably more than 80 wt%, and most preferably more than 90 wt%. An absorbent structure according to an embodiment of the invention has an increased loading capacity. An improved absorption capacity is made available.

In a second aspect, the present invention provides an absorbent article comprising, at least in the front half of the absorbent article, an absorbent structure according to an embodiment of an absorbent structure of the invention. It is known that for most absorbent articles, for instance for articles such as feminine hygiene garments, baby diapers, baby pants and adult incontinence products, liquid discharge occurs predominantly in the front half. It is therefore advantageous to provide an improved absorbing article according to an embodiment of the invention in that area where fluid uptake requirement is highest. Obviously, an absorbent article comprising an absorbent structure according to an embodiment of an absorbent structure of the invention which is entirely or partially located in either the front, crotch and/or back region of the absorbent article, such as for instance a baby diaper, is also covered under this invention.

In a preferred embodiment, the absorbent article has a crotch width of less than 90 mm. An absorbent article with a crotch width of this dimension provides improved consumer comfort. An absorbent article according to the invention achieves a crotch width of less than 85mm, 80mm, 75mm, 70 mm, 65 mm, 60 mm, 55 mm or 50 mm. Hence, preferably an absorbent structure according to the present invention has a crotch width as measured along a transversal line with which is positioned at equal distance to the front edge and the rear edge of the core which is of less than 100 mm, 90 mm, 80 mm, 70 mm, 60 mm or even less than 50 mm.

In a preferred embodiment, the absorbent structure is provided with a cover layer wherein portions of the cover layer are bonded or joined to portions of the substantially liquid-impermeable wicking layer via the immobilisation layer; the substantially liquid-impermeable wicking layer together with the cover layer thereby providing compartment or cavities for the clustering and immobilization and/or restraining the absorbent polymer material. Although not necessary to obtain the absorbent structure according to the invention, the ability to provide a formation of "valleys" in the cover layer and immobilisation layer provides an improved formability of the shape of the absorbent structure

In a preferred embodiment, the absorbent article as previously described, the portions of the cover layer bonded or joined to the portions of the substantially liquid-impermeable wicking layer provide areas of junction, said areas of junction are arranged along lines which form an angle of at least about 10 to 30 degrees with the longitudinal edges of the absorbent structure. The effect provided by the slightly declined areas of junction is that of a ply layer. It allows that the form of the absorbent article better matches that of the body of a subject. It provides more flexibility to the structure. Consequently improved comfort can be obtained.

In a preferred embodiment of the absorbent article of the invention, the upper and lower layers of the absorbent structure are attached to each other at both the front and back ends of the absorbent core.

In a third aspect, the present invention provides method for the manufacturing of an absorbent structure for use in an absorbent article comprising the steps of:
- providing a substantially liquid-impermeable wicking layer,
- covering the substantially liquid-impermeable wicking layer with an absorbent material wherein said absorbent material comprises
   i) an absorbent polymer material, and
   ii) from zero to an amount less than about 40 weight percent absorbent fibrous material, based on the weight of absorbent polymer material,
- covering the absorbent material with an immobilisation layer which is joinable to the substantially liquid-impermeable wicking layer, and
- joining the immobilisation layer to the substantially liquid-impermeable wicking layer to define compartments there between wherein the absorbent material is held in at least one of the compartments.

A substantially liquid-impermeable wicking layer as described above can be selected on the basis of material properties. Preferably the substantially liquid-impermeable wicking layer is provided in the form of a film or foil. The film or foil can be prepared by conventional techniques. It can be produced in large widths and lengths and cut to desired dimensions according to specifications required for the construction of the absorption structure. A film can for instance be obtained by extrusion or co-extrusion. To cover the absorbent material, an immobilisation layer is selected that is bondable or joinable to the substantially liquid-impermeable wicking layer. Bonding or joining the immobilisation layer to the substantially liquid-impermeable wicking layer provides compartments or pockets wherein absorbent material is enclosed. This enclosure of absorbent material inside pockets ensures that the absorbent material stays in place.

Preferably the bond or joint strength is obtained at a sealing temperature of at least the melting point of the easiest melting material of those materials one would like to bond together. Use of a low sealing temperature avoids that the absorption material enclosed in the pockets would be affected by the temperature developed for sealing. Suitable materials are for instance metallocene based polyethylene or polypropylene films. These films provide increased sealing speed in production lines.

A material not having the desired hydrophilicity may be provided with a coating layer. The coating layer can be applied by techniques known to a person skilled in the art. Prior to the application of a coating layer the film is preferably first corona treated.

In an embodiment of the method of the invention, the covering of the wicking material comprises applying to the substantially liquid-impermeable wicking layer a layer of a first absorbent polymer material followed by a layer of a second absorbent material, preferably absorbent polymer material wherein the particle size of the first absorbent material is smaller than the particle size of the second absorbent polymer material by at least about 10% to 50%. Using absorbent materials with different particle sizes is advantageous to obtain improved drainage in the layer comprising the absorbent materials.

In a preferred embodiment the substantially liquid-impermeable wicking layer is comprised of a polymer film, preferably a coated polyethylene (PE) film. The polyethylene film is preferably prepared by in-line coating, i.e. the coating is applied during the film production process prior to the longitudinal and/or transverse stretching process. In order to achieve good wetting of the polyethylene (PE) film by the aqueous coating composition, the surface is preferably first corona-treated. The coating can be applied by a commonly used suitable process, for example with a slot coater, or by a spray process. It is particularly preferable to apply the coating by the reverse gravure-roll process, which can apply an extremely homogeneous coating with application weights of from 0.5 to 5.0 g/m². The thickness of the coating on the finished film is preferably from 3 to 1000 nm, in particular from 30 to 200 nm.

During the drying and orientation of the polyethylene film, and particularly during the subsequent heat treatment the coating components can react with one another. The reaction product provides, specifically on a polyethylene film, a high level of hydrophilic properties.

The total thickness of the polyester film is usually in the range from 5 to 1500 µm, preferably from 10 to 500 µm.

In a more preferred embodiment, the substantially liquid-impermeable wicking layer is a corona-treated, hydrophilic, micro-embossed PE film in the 15-22 µm thickness range.

The substantially liquid-impermeable wicking layer is laid down onto a down drum, which presents a perforated surface containing openings in line with the preferred discrete locations of the absorbent materials comprising absorbent polymer materials. In a first process step the substantially liquid-impermeable wicking layer is laid on to the perforated surface and the absorbent polymer material is disposed by means know in the art. By using a vacuum means the absorbent polymer material will be drawn within the contours of the perforated vacuum means and thereby the absorbent polymer material will accumulate and cluster in predetermined locations according to the predefined perforation shapes. In a further process step an immobilisation layer is placed onto the absorbent polymer material. This immobilisation layer can be provided in the form of any suitable layer which can relatively immobilize the absorbent polymer materials once bonded or joined to the substantially liquid-impermeable wicking layer, ranging for example from any sort of fabrics or paper to glue and binders. In case of a non-inherent adhesive immobilisation layer, the immobilisation layer will need to be joined to the substantially liquid-impermeable wicking layer to obtain the compartments containing the clusters of absorbent material. If an inherent adhesive immobilisation layer, either with or without activation, is used the immobilisation will be automatically joined to the substantially liquid-impermeable wicking layer to obtain the compartments containing the clusters of absorbent material, although functional or complementary bonding by any means of the art (e.g. sonic bonding, adhesive, heat, pressure, etc) can be used to obtained the desired bonding strength along these junctions. While any immobilisation application means known in the art can be used to place the immobilisation layer onto the absorbent polymer material, in case of for instance a hot melt adhesive, the binder is preferably applied by a nozzle system. Preferably, a nozzle system is utilised which can provide a relatively thin but wide curtain of binder. This glue curtain can be continuous or discontinuous, so as to be applied in a homogeneous or heterogeneous surface or can be applied in various combinations of lines, grids, spirals, figures, spots, dots, etc. either in a determined or undetermined location of the target surface and/or any combination thereof. This curtain of adhesive is placed onto the substantially liquid-impermeable wicking layer and the absorbent polymer material so as to immobilise and/or compartmentalize the absorbent material. As the openings on the distribution and transport network are less covered by absorbent polymer material the binder will make contact with these areas of the substantially liquid-impermeable wicking layer and thereby capturing and immobilising the absorbent polymer materials.

In an optional further process step a cover layer is placed upon the substantially liquid-impermeable wicking layer, the absorbent polymer material and the immobilisation layer, for instance a hot melt adhesive layer. The cover layer will be bonded with the substantially liquid-impermeable wicking layer in the areas of junction by any means know in the art, without limitation for instance adhesives, sonic bonding, heat and/or pressure bonding, etc. In these areas of junction there is direct or indirect contact with the substantially liquid-impermeable wicking layer. The cover layer will typically not be in adhesive contact with the substantially liquid-impermeable wicking layer where the valleys of the distribution layer are filled with absorbent polymer material.

Alternatively the cover layer can be laid down onto a drum and the wicking network can be added in a consecutive process step.

In one alternative embodiment, the cover layer and the wicking network are provided from a unitary sheet of material. The placing of the cover layer onto the substantially liquid-impermeable wicking layer will then involve the folding of the unitary piece of material.

Hence, the uneven service of the lay-down system, which preferably is a lay-down drum, typically determines the distribution of absorbent polymer material throughout the absorbent material layer and likewise determines the pattern of areas of junction. Alternatively, the distribution of absorbent polymer material may be influenced by vacuum, air jet, conveyer surface, gravity, electrical or other means.

Preferably the distribution of absorbent polymer material is profiled and most preferably profiled in the longitudinal direction. Hence, along the longitudinal axis of the absorbent structure, which is normally coincident with the longitudinal axis of the absorbent article, for example of the baby diaper, the basis weight of the absorbent polymer material will change. Preferably the basis weight of absorbent polymer material in at least one freely selected first square measuring 1 cm x 1 cm is at least about 10 %, or 20%, or 30%, 40% or 50% higher than the basis weight of absorbent polymer material in at least one freely selected second square measuring 1 cm x 1 cm. Preferably the criterion is met if the first and the second square are centred about the longitudinal axis.

In a fourth aspect, an absorbing structure according to an embodiment of the invention is used as an absorbent core in a feminine hygiene garment, baby diaper, baby pants, or adult incontinence garment product.

In a preferred embodiment of a use of the invention, the substantially liquid-impermeable wicking layer comprises a polymer film, preferably polyethylene (PE) film, which has a hydrophilic coating on at least one surface. The use of this type of coated polyester film is advantageous as its high level of hydrophilic properties redirect liquid into versus the absorbing material. The anti-condensation action provided by this type of film is advantageous as it reduces the risk for leaks caused by liquid condensation and accumulation.

Upon review of the description of the invention and the accompanying drawings provided herein, it will be apparent to one of ordinary skill in the art that an absorbent structure made according to the present invention may be used in absorbent articles, and more particularly, in disposable absorbent articles, such as feminine hygiene garments, baby diapers and baby pants and adult incontinence garments.

Accordingly, the present invention shall not be limited to the article, structures and processes specifically described and illustrated herein, although the description provided was particularly directed to an absorbent disposable baby diaper product.

Examples are used below for further non-limitative illustration of the invention.

Preparation of an absorbent structure according to an embodiment of the invention

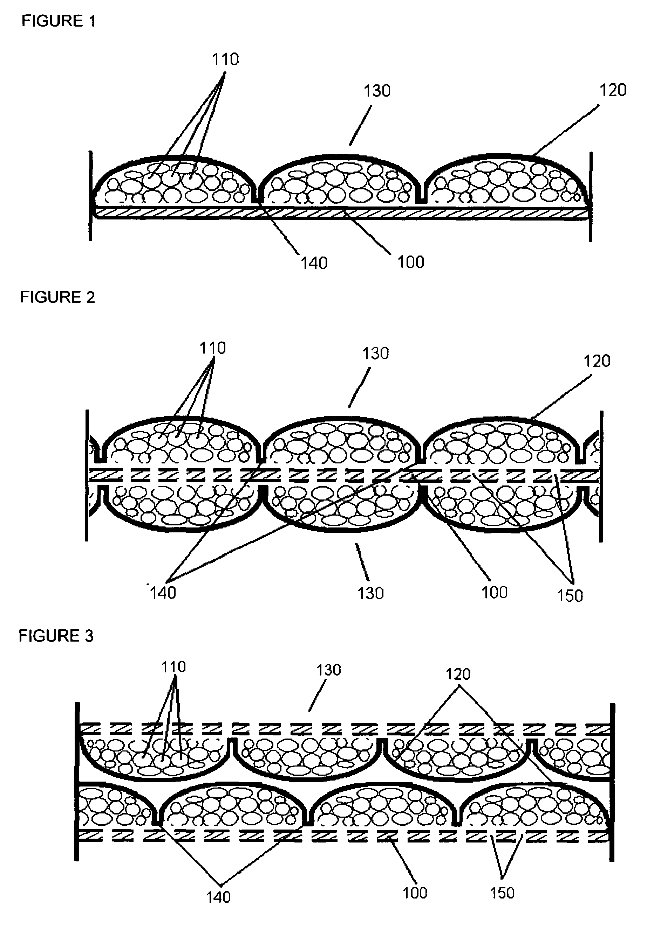

Referring to Figure 1, a substantially liquid-impermeable wicking layer 100 is provided. This substantially liquid-impermeable wicking layer 100 is having a contact angle with a water droplet lying thereon of less than about 30°. The substantially liquid-impermeable wicking layer 100 is covered on at least one side by discrete amounts of absorbent material 110. The absorbent material 110 is covered by an immobilisation layer 120. The immobilisation layer 120 lies on top of the absorbent material 110 and is joined at regular intervals by area's of junction 140 to the substantially liquid-impermeable wicking layer 100 thereby providing compartments 130 holding the absorbent material 110. The absorbent material 110 preferably comprises super absorbent polymer particles. In addition, absorbent fibrous materials can be used. Preferably the amount of absorbent fibrous material used is less than 20 weight percent based on the total weight of absorbent polymer material present.

Referring to Figure 2, it has been found that absorbent structures 14 can be formed by combining two layers of absorbent material 110. The absorbent structure 14 as shown in Figure 2 comprises one substantially liquid-impermeable wicking layer 100, two layers of absorbent material 110 and two immobilisation layers 120. When two discontinuous layers of an absorbent material 110 are used, being one on the wearer facing surface and one on the garment facing surface of the substantially liquid-impermeable wicking layer 100, they would be typically arranged in such a way that the compartments 130 containing the absorbent material 110 from one storage layer are aligned with the compartments 130 containing the absorbent material 110 from the other storage layer in order to have the area's of junction 140 from both layers adjacent to one another. In another alternative embodiment, however, the compartments 130 and the respective area's of junction 140 are offset one another. In a preferred embodiment of the invention the substantially liquid-impermeable wicking layer 100 is provided by openings 150 large enough to allow passage of liquid to ensure the liquid flow from one storage layer to the other storage layer. Such transport may for instance take place when the first storage layer is saturated with bodily fluids, while the second storage layer is still able to absorb additional liquids. In this way the absorbent structure 14 can lock away additional liquid at the garment facing side of the absorbent structure 14 below the substantially liquid-impermeable wicking layer 100 thereby further reducing the rewet values on the wearer facing surface.

Referring to Figure 3, it has also been found that absorbent structures 14 can be formed by combining two or more layers of absorbent material 110. The absorbent structure 14 as shown in Figure 3 comprises two substantially liquid-impermeable wicking layers 100, two layer of absorbent material 110 and two immobilisation layers 120. When two discontinuous layers of an absorbent material 110 are used, they would typically be arranged in such a way that the compartments 130 containing the absorbent material 110 of one storage layer faces the compartments 130 containing the absorbent material 110 of the other storage layer. In an alternative preferred embodiment, however, the area's of junction 140 are offset and do not face each other. Hence preferably, when two storage layers are joined, this is done such that the first immobilisation layer 120 of the first storage layer faces the immobilisation layer 120 of the second storage layer, while the two substantially liquid-impermeable wicking layers 100 from both storage layers are situated on the wearer facing surface and the garment facing surface of the combined absorbent structure 14. In a preferred embodiment of the invention the two substantially liquid-impermeable wicking layers 100 are provided by openings 150 large enough to allow passage of liquid to ensure the liquid flow to the storage layers. More preferably these openings 150 do not allow passage of the absorbent polymer material. The openings 150 can have various shapes, sizes and locations, they can be situated throughout the entire substantially liquid-impermeable wicking layer 100 or can be limited to parts thereof.

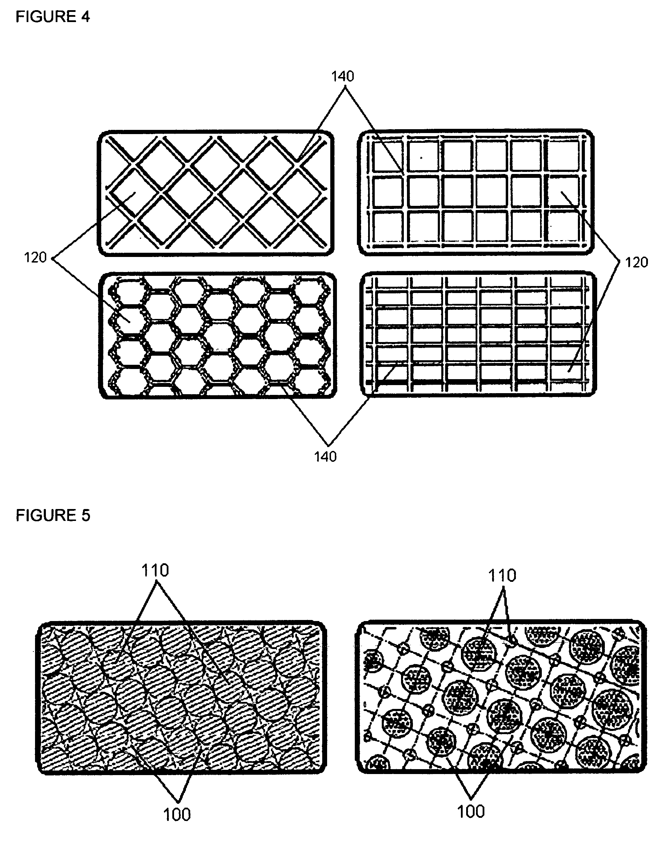

Referring to Figure 4, the areas of junction 140 can be disposed in different patterns. The lines of these patterns may be disposed regular or irregular, continuous or discontinuous, coextensive with the entire surface of the absorbent structure 14 or only part thereof and any combination and/or derivations thereof. These lines may be aligned with the longitudinal, transversal or diagonal axis of the absorbent structure 14 or alternatively any angle in respect of such axis. It has been found that, that a continuous disposition of these lines throughout the absorbent structure 14 creates channels and ducts which help the fluid transport from the point of liquid contact to the rest of the absorbent structure via mass flow allowing better and more spread out immediate fluid management via capillary flow locally. Another preferred pattern for areas of junction 140 is a pattern comprising polygons, for example pentagons and sexangles or a combination thereof. Also preferred are irregular patterns of area's of junction 140, which have been found to give good wet immobilisation of the absorbent material 110.

Referring to Figure 1E, the clusters of absorbent material 110 can be disposed in different patterns, sizes and locations. The disposition may be regular or irregular, continuous or discontinuous, covering the entire surface of the absorbent structure or only part thereof and any combination and derivations thereof.

Preparation of an absorbent article according to an embodiment of the invention

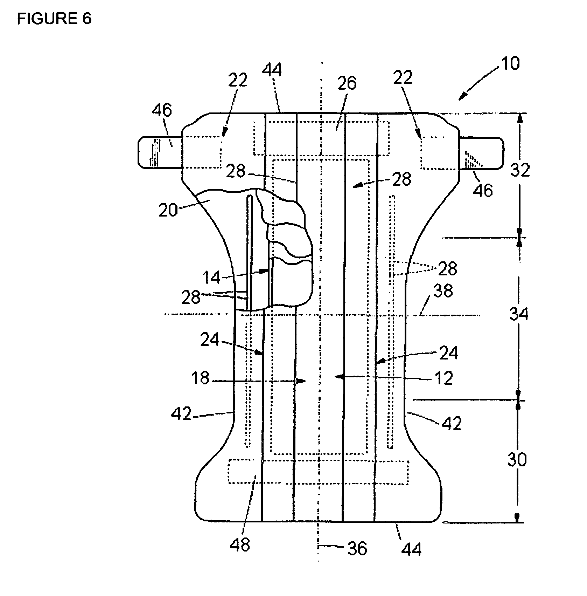

Figure 6 is a top plan view of a diaper 10 as a preferred embodiment of an absorbent article including an absorbent structure according to the present invention. It should be understood, however, that the present invention is also applicable to other absorbent articles such as feminine hygiene garments, baby pants, adult incontinent garments and the like.

The absorbent article is shown in its flat out, un-contracted state with the wearer side facing the viewer. Portions of the absorbent article are cut away to more clearly show the underlying structure of the diaper 10 including the absorbent elements and absorbent components. The chassis 12 of the diaper 10 in Figure 6 comprises the main body of the diaper 10. The chassis 12 comprises an outer covering including a liquid pervious top sheet 18 and/or a liquid impervious back sheet 20. The chassis 12 may include a portion of an absorbent structure 14 encased between the top sheet 18 and the back sheet 20. The chassis 12 may also include most or all of the absorbent structure 14 encased between the top sheet 18 and the back sheet 20. The chassis 12 preferably further includes side panels or ears 22, elasticized leg cuffs 24 and elastic waist features 26, the leg cuffs 24 and the elastic waist feature 26 each typically comprise elastic members 28. One end portion of the diaper 10 is configured as a front waist region 30 of the diaper 10. The opposite end portion is configured as a back waist region 32 of the diaper 10. An intermediate portion of the diaper 10 is configured as a crotch region 34, which extends longitudinally between the first and second waist regions 30 and 32. The waist regions 30 and 32 may include elastic elements such that they gather about the waist of the wearer to provide improved fit and containment (e.g. elastic waist feature 26). The crotch region 34 is that portion of the diaper 10 which, when the diaper 10 is worn, is generally positioned between the wearer's legs. The diaper 10 is depicted with its longitudinal axis 36 and its transverse axis 38. The periphery of the diaper 10 is defined by the outer edges of the diaper 10 in which the longitudinal edges 42 run generally parallel to the longitudinal axis 36 of the diaper 10 and the end edges 44 run between the longitudinal edges 42 generally parallel to the transverse axis 38 of the diaper. The chassis 12 also comprises a fastening system, which may include at least one fastening or securing member 46 and at least one landing zone 48. The various components within the diaper 10 may be bound, joined or secured by any method know in the art, for example by adhesives in uniform continuous layers, patterned layers or arrays of separate lines, spirals or spots. The top sheet 18, the back sheet 20, the absorbent structure 14 and other components may be assembled in a variety of well-known configurations and are well known in the art.

The back sheet 20 covers the absorbent structure 14 and preferably extends beyond the absorbent structure 14 toward the longitudinal edges 42 and end edges 44 of the diaper 10 and may be joined with the top sheet 18. The back sheet 20 prevents the bodily exudates absorbed by the absorbent structure 14 and contained within the diaper 10 from soiling other external articles that may contact the wearer, such as bed sheets and undergarments. In preferred embodiments, the back sheet 20 is substantially impervious to bodily exudates and comprises a laminate of a non-woven and a thin plastic film such as a thermoplastic film. The back sheet 20 may comprise breathable materials that permit vapour to escape from the diaper 10 while still preventing bodily exudates from passing through the back sheet 20. It may be semi-rigid, non-elastic and can be made fully or partially elasticized and include backing. The back sheets 20 may be assembled in a variety of well-known configurations and are well known in the art.

The diaper 10 comprises a top sheet 18 that is preferably soft, compliant, exhibits good strikethroughs and has a reduced tendency to rewet from the liquid absorbent material. The top sheet 18 is placed in close proximity to the skin of the wearer when the diaper 10 is worn. In this way, such top sheet 18 permits bodily exudates to rapidly penetrate it so as to flow toward the absorbent structure 14 more quickly, but preferably not allowing such bodily exudates to flow back through the top sheet 18. The top sheet 18 may be constructed from any one of a wide range of liquid and vapour permeable, preferably hydrophilic, materials. The upper and lower surface of the top sheet 18 may be treated differently and may for instance include a surfactant on the upper surface so as to facilitate liquid transfer there through, especially at a central zone or area of the top sheet 18 located over the absorbent structure 10, and for instance include a hydrophobic agent on the lower surface to minimize the liquid contained within the absorbent core from contact wetting the top sheet 18 thereby reducing rewet values. The top sheet 18 may also be coated with a substance having rash preventing or rash reducing properties (e.g. aloe vera). The top sheet 18 covers substantially the entire wearer facing area of the diaper 10, including substantially all of the front waist region 30, back waist region 32, and crotch region 34. Further, the side panels 22 and/or waist feature layers of the inner region may be formed from the same single top sheet material and, thus, may be referred to as being unitary with the top sheet 18 in forming longitudinal and lateral extensions of the top sheet 18 material. Alternatively, the top sheet 18 may be formed from multiple different materials which vary across the width of the top sheet 18. Such a multiple piece design allows for creation of preferred properties and different zones of the top sheet 18. The top sheet 18 be semi-rigid, non-elastic and can be made fully or partially elasticized. The top sheet 18 may be assembled in a variety of well-known configurations and are well known in the art.

The absorbent structure 14 in Figure 6 generally is disposed between the top sheet 18 and the back sheet 20. The absorbent structure 14 may comprise any absorbent material 110 that is generally compressible, conformable, non-irritating to the wearer's skin, and capable of absorbing and retaining bodily exudates. The absorbent structure 14 may comprise a wide variety of liquid absorbent materials 110 commonly used in absorbent articles such as fluff pulp, which is generally referred to as airlaid. Examples of other suitable absorbent materials include creped cellulose wadding; melt blown polymers; chemically stiffened, modified or cross-linked cellulosic fibres; tissue, including tissue wraps and tissue laminates; absorbent foams; absorbent sponges; absorbent polymer materials; absorbent gelling materials; or any other known absorbent materials or combinations of materials. The absorbent structure 14 may further comprise minor amounts (typically less than 10 %) of non-liquid absorbent materials, such as adhesives, binders, plastics, waxes, oils and the like. The absorbent structure 14 according to various embodiments of the invention may be configured to extend substantially the full length and/or width of the diaper 10. However, alternatively the absorbent structure 14 according to the invention is not coextensive with the entire diaper 10 and is limited to certain regions of the diaper 10 such as for instance the crotch region 34. In various embodiments, the absorbent structure 14 extends to the edges of the diaper 10 and the absorbent material 110 is concentrated in the crotch region 34 or another target zone of the diaper 10. In still another embodiment, the particles can be a combination of absorbent material 110, preferably comprising absorbent polymer material, and skin care particles such as ion exchange resins, deodorant, anti-microbial agents, binder particles, or other beneficial particles.

The diaper 10 may also utilize a pair of containment walls or cuffs 24. Each cuff 24 is a longitudinally extending wall structure preferably positioned on each side of the absorbent structure 14 and spaced laterally from the longitudinal axis 36. The longitudinal ends of the cuffs 24 may be attached or joined, for example, to the top sheet 18 in the front and rear waist regions 30 and 32. Preferably, the ends of the cuffs 24 are tacked down inwardly and attached, for example, by adhesive or sonic bonding to the lower structure. Such a construction effectively biases the cuffs 24 inwardly and is generally considered to cause the cuffs 24 to exhibit improved leakage prevention properties. Preferably, the cuffs 24 are equipped with elastic members 28, which extend along a substantial length of the cuffs 24. In a common application, the elastic members 28 are placed within the cuffs 24, preferably at the top of the cuff 24 while in a stretched condition and then glued or sonic bonded to the cuff 24 at least at their ends. When released or otherwise allowed relaxing, the elastic members 28 retract inwardly. When the diaper 10 is worn, the elastic members 28 function to contract the cuffs 24 about the buttocks and the thighs of the wearer in a manner, which forms a seals between the diaper 10, the buttocks and the thighs. The cuffs 24 may be assembled in a variety of well-known configurations and are well known in the art.

The diaper 10 may also employ additional layers known in the art including an acquisition layer or surge layer, preferably situated between the top sheet and the absorbent core and highloft and/or coverstock layers. This serves to slow down the flow so that the liquid has adequate time to be absorbed by the absorbent core.

In order to keep the diaper 10 in place about the wearer, preferably at least a portion of the back waist region 32 is attached by fastening or securing members 46 to at least a portion of the front waist region 30, preferably to form leg openings and an absorbent article waist. Fastening or securing members 46 carry the tensile load around the absorbent article waist and compliment the elastic members 28 by providing a quasi-seal between the wearer, the elastic waist feature 26 and cuffs 24, so that bodily exudates are contained within the diaper 10 which are then absorbed. In other words, so that it does not leak through gaps between the wearer and the edge of the diaper 10. The fastening or securing members 46 may for instance be adhesive, mechanical fasteners, hook and loop features, conceivable strings and/or combinations thereof, i.e., anything that will secure one end of the diaper 10 to the longitudinally opposite end of the diaper 10. The fastening or securing members 46 may also be co-adhesive such that they adhere to each other but not other materials. The fastening or securing members 46 and any component thereof may include any material suitable for such a use, including but not limited to plastics, films, foams, non-woven webs, woven webs, paper, laminates, fibre reinforced plastics and the like, or combinations thereof. It may be preferable that the materials making up the fastening or securing members 46 are flexible, extensible and/or elastic, allowing them to better conform to the shape and movements of the body and thus, reduces the likelihood that the fastening system will irritate or injure the wearer's skin. Preferably, the diaper 10 is affixed to the wearer by tape fasteners which are permanently affixed to the back sheet 20. Tape fasteners are contacted with the transversely opposite side panel or ears 22 attached or joined and extending from the back sheet 20, where they remain affixed due to the binding compound applied to the fasteners. Alternatively, the absorbent article may be pants and the like. In this configuration, the absorbent article may or may not have tape fasteners. Specific disposability tapes may however also be provided on such absorbent articles. All fastening and securing elements 46 may be assembled in a variety of well-known configurations and are well known in the art.

The waist regions 30 and 32 each comprise a central region and a pair of side panels or ears 22 which typically comprise the outer lateral portions of the waist regions. These side panels 22 may be unitary with the chassis 12 and/or back sheet 20 or may be attached or joined thereto by any means know in the art. In a preferred embodiment of the present invention, the side panels 22 positioned in the back waist region 32 are flexible, extensible and/or elastic in at least the lateral direction (i.e., elasticized side panels), in another embodiment the side panels 22 are non-elastic, semi-rigid, rigid and/or stiff. This variety of side panels 22 are well known in the art.

Furthermore waistbands 26 employing elastic members can be positioned along the transverse portion of the diaper 10 so that when worn, the waistbands 26 are positioned along the waist of the wearer. Generally, the waistband 26 preferably creates a seal against the waist so that bodily exudates do not leak from the regions between the elastic waistband 26 and the waist of the wearer. Although the bodily exudates are primarily absorbed by the absorbent materials within the diaper 10, the seal is important considering the assault of liquid by the wearer may overwhelm the absorption rate capacity of the absorbent structure 14. Hence, the waistbands 26 contain the liquid while it is being absorbed, they are well known in the art.

The absorbent article such as a diaper 10 may also include such other features, components and elements as are known in the art including front and rear ear panels, waist cap features, elastics and the like to provide better fit, containment and aesthetic characteristics. These features may be assembled in a variety of well-known configurations and are well known in the art.

## Claims

1. An absorbent structure for use in an absorbent article comprising:
a) a substantially liquid-impermeable wicking layer and
b) an immobilisation layer which is joined to the substantially liquid-impermeable wicking layer to define compartments there between; and
c) an absorbent material held in at least one of the compartments:
i) wherein said absorbent material comprises an absorbent polymer material, and
ii) from zero to an amount less than about 40 weight percent absorbent fibrous material, based on the weight of absorbent polymer material.

2. Absorbent structure according to claim 1, wherein the substantially liquid-impermeable wicking layer is a substantially continuous layer of film, foil, plastic or closed foam.

3. Absorbent structure according to claim 2, wherein said film, foil, plastic or closed foam is provided with a hydrophilic or hydrophobic coating or combination thereof.

4. Absorbent structure according to claim 2 or 3, wherein said film, foil, plastic or closed foam is provided with access means, such as perforations to a second layer of absorbent material.

5. Absorbent structure according to any of claims 1 to 4, wherein the immobilisation layer is a thermoplastic adhesive, non-woven, polymer film, paper, tissue, airlaid, drylaid or wet laid material.

6. Absorbent structure according to any of claims 1 to 5, wherein the joints between the lower surface of the immobilisation layer and the upper surface of the wicking layer are not permanent so that the bonds partially loosen during wetting.

7. Absorbent structure according to any of claims 1 to 6, wherein the immobilisation layer bonded to the substantially liquid-impermeable wicking layer has a dry bond strength of at least 0.05 N/cm.

8. Absorbent structure according to any of claims 1 to 7, wherein the absorbent material comprises an absorbent polymer material, preferably the absorbent polymer material is present in the absorbent material in an average basis weight of at least 100 g/m².

9. Absorbent structure according to any of claims 1 to 8, wherein the absorbent structure has a flexure-resistance of less than 500.0 grams, more preferably less than 250.0 grams, and still more preferably less than about 175.0 grams and most preferably less than 130.0 grams or 100.0 grams.

10. Absorbent structure according to any of claims 1 to 9, wherein the wet immobilization is more than 50 wt%.

11. An absorbing article comprising, at least in the front half of the absorbing article, an absorbent structure according to any of the above claims 1 to 10.

12. Absorbing article according to claim 11, wherein the absorbent structure is provided with a cover layer wherein portions of the cover layer are joined to portions of the substantially liquid-impermeable wicking layer via the immobilisation layer; the substantially liquid-impermeable wicking layer together with the cover layer thereby providing cavities for the immobilization of the absorbent polymer material.

13. Absorbing article according to claim 12, wherein the portions of the cover layer joined to the portions of the substantially liquid-impermeable wicking layer provide areas of junction, said areas of junction are arranged along lines which form an angle of at least 15 degrees with the longitudinal edges of the absorbent structure.

14. Method for the manufacturing of an absorbent structure for use in an absorbent article comprising the steps of:
- providing a substantially liquid-impermeable wicking layer,
- covering the substantially liquid-impermeable wicking layer with an absorbent material wherein said absorbent material comprises
i) an absorbent polymer material, and
ii) from zero to an amount less than about 40 weight percent absorbent fibrous material, based on the weight of absorbent polymer material,
- covering the absorbent material with an immobilisation layer which is joinable to the substantially liquid-impermeable wicking layer, and
joining the immobilisation layer to the substantially liquid-impermeable wicking layer to define compartments there between wherein the absorbent material is held in at least one of the compartments.

15. Method according to claim 14, wherein the covering of the wicking material comprises applying to the substantially liquid-impermeable wicking layer a layer of a first absorbent polymer material followed by a layer of a second absorbent polymer material, wherein the particle size of the first absorbent material is smaller than the particle size of the second absorbent polymer material by at least 5%.
